# EUROPEAN PATENT APPLICATION

(11) **EP 2 642 419 A2**
(43) Date of publication of application: **25.09.2013**
(21) Application number: 13160609.7
(22) Date of filing: 22.03.2013
(51) Int. Cl.: G06F 19/00

(54) **Method and apparatus for providing data to user of ultrasound apparatus**

(30) Priority: 23.03.2012 KR 20120030029
(71) Applicant: Samsung Medison Co., Ltd., Gangwon-do 250-870 (KR)
(72) Inventor: Choi, Young-min, Gangwon-do (KR)
(74) Representative: Lorenz, Markus

(57) **Abstract**

A method of providing data to a user of an ultrasound apparatus, the method including: transmitting identification information input by the user to an external server; receiving data including use environment information of the ultrasound apparatus, which corresponds to the identification information, from the external server; and changing use environment of the ultrasound apparatus according to the received use environment information.

## Description

### CROSS-REFERENCE TO RELATED PATENT APPLICATION

This application claims the benefit of Korean Patent Application No. 10-2012-0030029, filed on March 23, 2012, in the Korean Intellectual Property Office, the disclosure of which is incorporated herein in its entirety by reference.

### BACKGROUND OF THE INVENTION

### 1. Field of the Invention

The present invention relates to a method and apparatus for providing data to a user of the ultrasound apparatus, and more particularly, to a method and apparatus for providing data to a user of the ultrasound apparatus based on identification information of the user.

### 2. Description of the Related Art

An ultrasound apparatus is used for observing an internal structure of an organic body. The ultrasound apparatus, which is a noninvasive diagnosis apparatus, shows structural details, an internal organization, and a flow of fluid in the human body.

The ultrasound apparatus transmits an ultrasound signal to a target object through the human body, and obtains an image of an internal structure of the human body by receiving an echo signal from the target object.

An ultrasound image obtained from an examinee is stored in a storage unit including a hard disk in an ultrasound apparatus, but the image may be lost if an error occurs in the storage unit of the ultrasound apparatus.

In addition, a user of an ultrasound apparatus sets use environment of the ultrasound apparatus according to his or her own taste or convenience, but, when the user uses a new ultrasound apparatus instead of the ultrasound apparatus of which the use environment has been previously set by the user, the user has to set again use environment of the new ultrasound apparatus.

### SUMMARY OF THE INVENTION

The present invention provides a method and apparatus for providing data to a user of an ultrasound apparatus, in which ultrasound images obtained by using the ultrasound apparatus are stored in an external server in correspondence to each of users to thus enable a user to easily obtain ultrasound images which the user has previously captured.

The present invention also provides an apparatus and method for providing data to a user of an ultrasound apparatus, in which previously set use environment may be applied to another ultrasound apparatus although the user does not use the previously used ultrasound apparatus but uses another ultrasound apparatus.

According to an aspect of the present invention, there is provided a method of providing data to a user of an ultrasound apparatus, the method including: transmitting identification information input by the user to an external server; receiving data including use environment information of the ultrasound apparatus, which corresponds to the identification information, from the external server; and changing use environment of the ultrasound apparatus according to the received use environment information.

The method may further include transmitting changed use environment information to the external server when the use environment of the ultrasound apparatus is changed by the user.

The use environment information may include at least one of information about a method of displaying an image obtained by the ultrasound apparatus, information about a method of calculating values measured by the ultrasound apparatus, and information about an user interface (UI) of the ultrasound apparatus.

The data may further include medical examination information of an examinee.

The medical examination information may include at least one of information about the examinee, an ultrasound image of the examinee, and a measured value obtained from the ultrasound image.

According to another aspect of the present invention, there is provided a method of providing data to a user of an ultrasound apparatus, the method including: receiving identification information of a user from the ultrasound apparatus; and transmitting data including use environment information of the ultrasound apparatus, which corresponds to the received identification information, to the ultrasound apparatus.

The method may further include: receiving changed use environment information of the ultrasound apparatus from the ultrasound apparatus; and storing the changed use environment information in correspondence to the identification information of the user.

According to another aspect of the present invention, there is provided an apparatus for providing data to a user of an ultrasound apparatus, the apparatus including: a transmission unit for transmitting identification information input by the user to an external server; a reception unit for receiving data including use environment information of the ultrasound apparatus, which corresponds to the identification information, from the external server; and a control unit for changing use environment of the ultrasound apparatus according to the received use environment information.

The apparatus may further include a sensing unit for sensing whether the use environment of the ultrasound apparatus has been changed by the user, wherein the transmission unit transmits changed use environment informaition of the ultrasound apparatus to the external server.

The use environment information may include at least one of information about a method of displaying an image obtained by the ultrasound apparatus, information about a method of calculating values measured by the ultrasound apparatus, and information about an user interface (UI) of the ultrasound apparatus.

The data may further include medical examination information of an examinee.

The medical examination information may include at least one of information about the examinee, an ultrasound image of the examinee, and a measured value obtained from the ultrasound image.

According to another aspect of the present invention, there is provided a server for providing data to a user of an ultrasound apparatus, the server including: a reception unit for receiving identification information of a user from the ultrasound apparatus; and a transmission unit for transmitting data including use environment information of the ultrasound apparatus, which corresponds to the received identification information, to the ultrasound apparatus.

The server may further include a control unit for storing changed use environment information of the ultrasound apparatus by matching it to the identification information of the user when the reception unit receives the changed use environment information from the ultrasound apparatus.

According to another aspect of the present invention, there is provided a computer-readable recording medium storing a computer-readable program for executing the method of providing data to a user of an ultrasound apparatus.

### BRIEF DESCRIPTION OF THE DRAWINGS

The above and other features and advantages of the present invention will become more apparent by describing in detail exemplary embodiments thereof with reference to the attached drawings in which:
FIG. 1A is a block diagram illustrating a configuration of an ultrasound apparatus according to an embodiment of the present invention;
FIG. 1B is a block diagram illustrating a configuration of an ultrasound apparatus according to another embodiment of the present invention;
FIG. 2A is a block diagram illustrating a configuration of a server according to an embodiment of the present invention;
FIG. 2B is a block diagram illustrating a configuration of a server according to another embodiment of the present invention;
FIG. 3 is a diagram illustrating a process in which data is transmitted between an ultrasound apparatus and a server, according to an embodiment of the present invention;
FIG. 4 is a flowchart illustrating a method of providing data, according to an embodiment of the present invention; and
FIG. 5 is a flowchart illustrating a method of providing data, according to another embodiment of the present invention.

### DETAILED DESCRIPTION OF THE INVENTION

The merits and characteristics of the present invention and methods for achieving them will be clear with reference to the embodiments described below in detail with the attached drawings. However, the present invention is not limited to the embodiments disclosed below, and the present invention will be implemented in various forms; rather, these embodiments are provided so that this disclosure will be thorough and complete, and will fully convey the concept of the invention to those of ordinary skill in the art. The present invention is only defined by the following claims. Like reference numbers are used to refer to like elements throughout the specification.

The term 'unit' used in the embodiments indicates a software or hardware component, such as a Field Programmable Gate Array (FPGA) or Application Specific Integrated Circuit (ASIC), and performs a certain role. However, the term 'unit' is not limited to software or hardware. The term 'unit' may be configured to be in an addressable storage medium or to reproduce one or more processors. Thus, for example, the term 'unit' includes components, such as software components, object-oriented software components, class components, and task components, processors, functions, attributes, procedures, sub-routines, program code segments, drivers, firmware, micro-codes, circuits, data, databases, data structures, tables, arrays, and variables. A function provided in components and 'units' may be performed by combining a smaller number of components and 'units' or further separating additional components and 'units' therefrom.

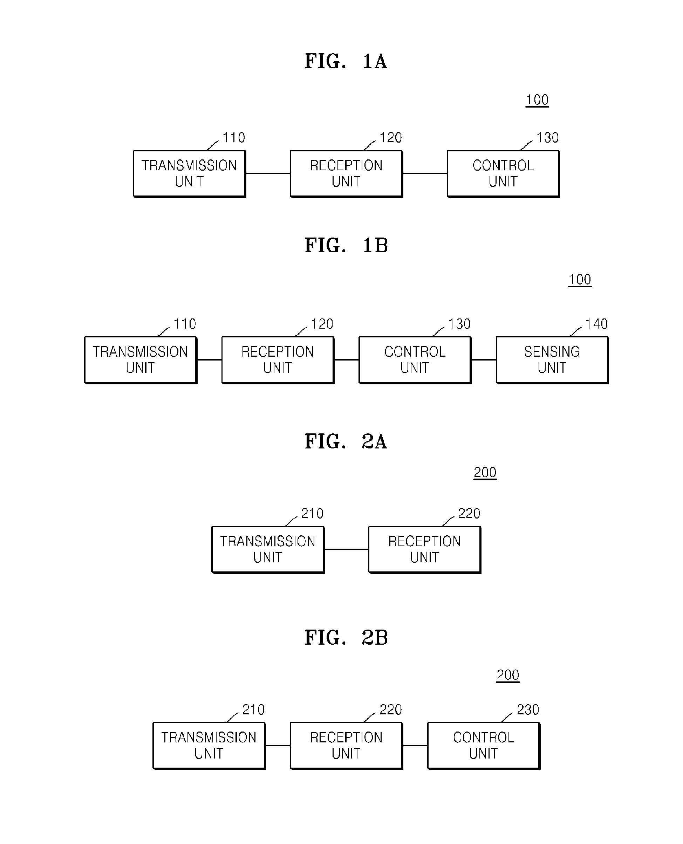

FIG. 1A is a block diagram illustrating a configuration of an ultrasound apparatus 100 according to an embodiment of the present invention.

Referring to FIG. 1A, the ultrasound apparatus 100 may include a transmission unit 110, a reception unit 120, and a control unit 130. The control unit 130 may be implemented by using a micro chip.

When identification information of a user is input to the ultrasound apparatus 100 by a user, the transmission unit 110 transmits corresponding information to an external server.

The identification information may be ID (Identification) or a password of a user of the ultrasound apparatus 100, and commonly known identification information including a user's fingerprint by which a user may be identified may be input to the ultrasound apparatus 100. The external server may be physically separated from the ultrasound apparatus 100, and may transmit or receive data to or from the ultrasound apparatus 100 through wired communication or wireless communication.

Identification information of users of the ultrasound apparatus 100 is stored in the external server, and the external server matches identification information received from the transmission unit 110 with previously stored identification information.

The external server transmits data corresponding to the identification information received from the transmission unit 110 to the ultrasound apparatus 100, and the reception unit 120 of the ultrasound apparatus 100 receives the data.

The data that is transmitted from the external server includes information about use environment (hereinafter, referred to as "use environment information") of the ultrasound apparatus 100. The use environment information may include at least one of information about a method of displaying an image obtained by the ultrasound apparatus 100, information about a method of calculating values measured by the ultrasound apparatus 100, and information about an user interface (UI) of the ultrasound apparatus 100.

The information about a method of displaying an image obtained by the ultrasound apparatus 100 is information about what method of displaying examinee's ultrasound images captured by using the ultrasound apparatus 100. For example, the information about a method of displaying may include information about brightness and contrast of an ultrasound image, a method of expressing curves, or colors.

The information about a method of calculating values measured by the ultrasound apparatus 100 may include whether to calculate the values measured from the ultrasound image by using what formula or statistical data or whether to set an approximate value.

The information about an UI of the ultrasound apparatus 100 may include a configuration of a menu that is expressed on a display of the ultrasound apparatus 100, kinds of information that is expressed by ultrasound images, or the like.

In addition, the information about a method of displaying an image obtained by the ultrasound apparatus 100, the information about a method of calculating values measured by the ultrasound apparatus 100, and the information about an UI (User Interface) of the ultrasound apparatus 100 may include commonly known other information.

The control unit 130 changes use environment of the ultrasound apparatus 100 according to received use environment information.

The use environment of the ultrasound apparatus 100 may be set in various forms according to taste or convenience of users, and the ultrasound apparatus 100 according to an embodiment of the present invention may solve a problem that a user should manually set again the use environment, by receiving use environment information, which the user desires to set, from an external server, although the user uses any other ultrasound apparatus 100.

FIG. 1B is a block diagram illustrating a configuration of an ultrasound apparatus 100 according to another embodiment of the present invention.

Referring to FIG. 1B, the ultrasound apparatus 100 according to another embodiment of the present invention may further include a sensing unit 140 when compared to the ultrasound apparatus 100 illustrated in FIG. 1A. Since a transmission unit 110, a reception unit 120, and a control unit 130 of FIG. 1B are the same as those of FIG. 1A, a detailed description thereof is omitted.

The sensing unit 140 senses whether use environment of the ultrasound apparatus 100 has been changed by a user. In detail, the sensing unit 140 senses whether the use environment of the ultrasound apparatus 100 has been newly changed by a user of the ultrasound apparatus 100 by using use environment information received from an external server.

The transmission unit 110 transmits changed use environment information of the ultrasound apparatus 100 to the external server. A method of transmitting the changed use environment information to the external server by the transmission unit 110 may be variously set. Whenever use environment information is changed, the changed use environment information may be transmitted to the external server. Also, the changed use environment information may be transmitted to the external server by predetermined input of a user, and, when a user logs out of the ultrasound apparatus 100, changed use environment information may be transmitted to the external server. Thus, the external server may update use environment information.

Data which the ultrasound apparatus 100 receives from the external server may include medical examination information of an examinee.

The medical examination information may include at least one of information about the examinee, an ultrasound image of the examinee, and a measured value obtained from the ultrasound image.

The information about the examinee may include personal information for identifying the examinee or research information about the examinee.

The ultrasound image of the examinee includes a two dimensional (2D) image, a three dimensional (3D) image, or a moving image, as an ultrasound image captured for the examinee.

The measured value obtained from the ultrasound image includes a length or width of an object directly obtained from an ultrasound image of the examinee or a value derived by using a measured value directly obtained from the ultrasound image.

In addition, the information about the examinee, the ultrasound image of the examinee, and the measured value obtained from the ultrasound image may include commonly known other information.

Since the ultrasound apparatus 100 provides medical examination information of an examinee to a user, the user may easily manage an ultrasound image of the examinee, which is captured by the user.

The transmission unit 110 may also transmit newly obtained medical examination information of an examinee to the external server. A method of transmitting the newly obtained medical examination information of an examinee to the external server by the transmission unit 110 may be variously set. Whenever medical examination information of an examinee is newly obtained, the newly obtained medical examination information may be transmitted to the external server. Also, the newly obtained medical examination information may be transmitted to the external server by predetermined input of a user, and when a user logs out of the ultrasound apparatus 100, obtained medical examination information may be transmitted to the external server. Thus, the external server may update medical examination information of an examinee depending on a user.

The ultrasound apparatus 100 according to the current embodiment of the present invention may improve continuity of operation by displaying again an image which a user has previously displayed, if the user inputs identification information to the ultrasound apparatus 100.

FIG. 2A is a block diagram illustrating a configuration of a server 200 according to an embodiment of the present invention.

Referring to FIG. 2A, the server 200 may include a transmission unit 210 or a reception unit 220.

The reception unit 220 receives identification information of a user from the ultrasound apparatus 100.

The transmission unit 210 transmits data including use environment information of the ultrasound apparatus 100, which corresponds to the received identification information, to the ultrasound apparatus 100.

Data which the transmission unit 210 transmits to the ultrasound apparatus 100 may further include medical examination information of an examinee.

FIG. 2B is a block diagram illustrating a configuration of a server 200 according to another embodiment of the present invention.

Referring to FIG. 2B, the server 200 according to another embodiment of the present invention further includes a control unit 230 compared to the server 200 illustrated in FIG. 2A.

When a user changes use environment of the ultrasound apparatus 100, the ultrasound apparatus 100 transmits changed use environment information of the ultrasound apparatus 100 to the server 200. Then, when the reception unit 220 receives the changed use environment information, the control unit 230 stores the changed use environment information in correspondence to identification information of a user.

In addition, when newly obtained medical examination information of an examinee is received from the ultrasound apparatus 100, the control unit 230 may store the newly obtained medical examination information in correspondence to identification information of a user.

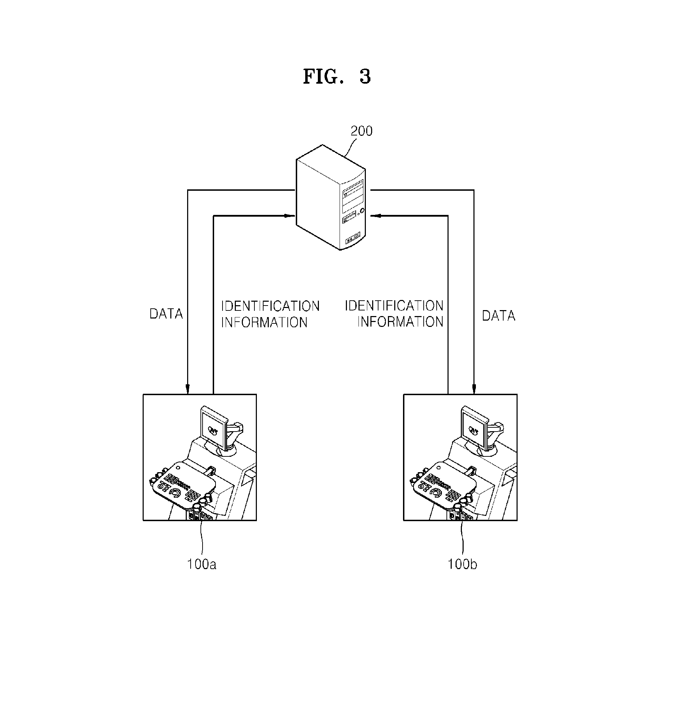

FIG. 3 is a diagram illustrating a process in which data is transmitted between the ultrasound apparatus 100a or 100b and the server 200, according to an embodiment of the present invention.

When identification information of a user is input through the ultrasound apparatus 100a or 100b, the identification information is transmitted to the server 200, i.e., an external server, and the server 200 transmits data corresponding to the identification information to the ultrasound apparatus 100a or 100b.

Although a user uses another ultrasound apparatus 100b after using one ultrasound apparatus 100a, the user may work under the same use environment in the ultrasound apparatuses 100a and 100b, and continuity of operation may be improved through the same medical examination information.

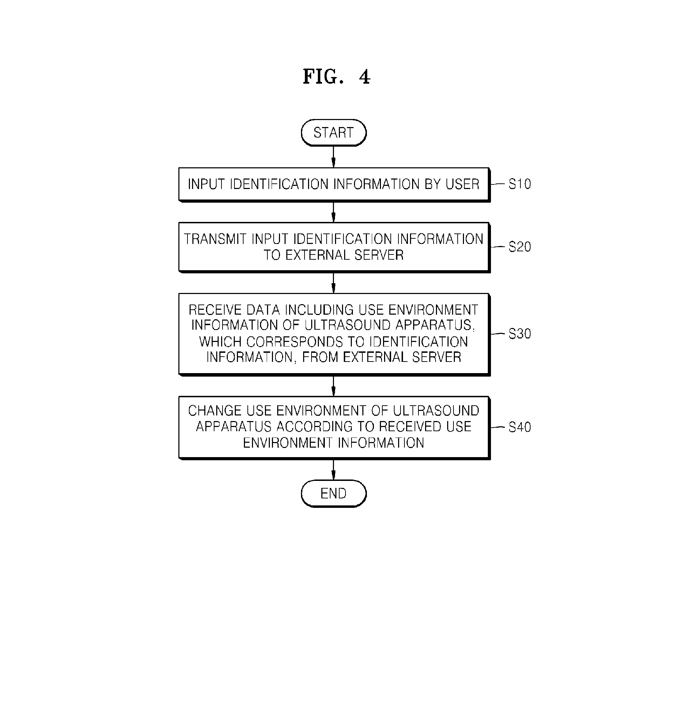

FIG. 4 is a flowchart illustrating a method of providing data, according to an embodiment of the present invention.

Referring to FIG. 4, the method of providing data includes operations that are sequentially processed in the ultrasound apparatus 100 illustrated in FIG. 1A or 1B. Thus, although omitted below, contents described above with respect to the ultrasound apparatus 100 illustrated in FIG. 1A or 1B may be applied to the data providing method of FIG. 4.

In operation S10, identification information of a user is input to the ultrasound apparatus 100 by the user. The identification information may be input by using a keyboard, a mouse, a fingerprint recognition apparatus, etc.

In operation S20, the ultrasound apparatus 100 transmit the input identification information to the external server 200.

In operation S30, the ultrasound apparatus 100 receives data including use environment information of the ultrasound apparatus 100, which corresponds to the identification information, from the external server 200. As stated above, the data received from the external server 200 may include medical examination information of an examinee.

In operation S40, the ultrasound apparatus 100 changes use environment of the ultrasound apparatus 100 according to received use environment information. In addition, the ultrasound apparatus 100 may continuously display medical examination information that has been previously displayed to a user, when the user inputs the identification information.

When the use environment of the ultrasound apparatus 100 is changed by a user or new medical examination information is obtained, the ultrasound apparatus 100 may transmit the changed use environment information or the new medical examination information to the external server 200 to update corresponding information at the external server 200.

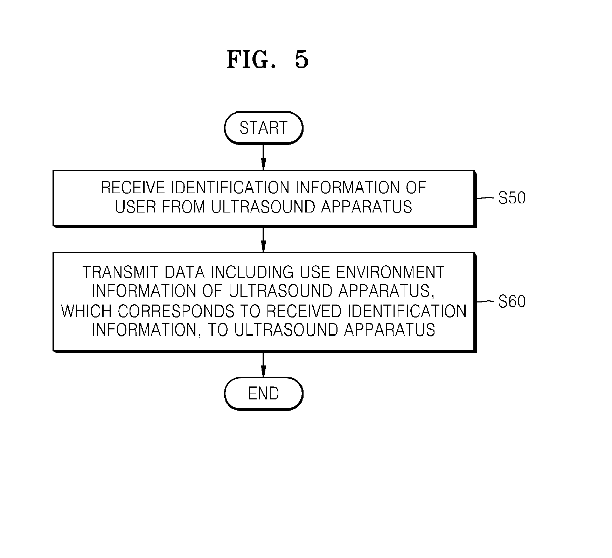

FIG. 5 is a flowchart illustrating a method of providing data, according to another embodiment of the present invention.

Referring to FIG. 5, the method of providing data includes operations that are sequentially processed in the server 200 illustrated in FIG. 2A or 2B. Thus, although omitted below, contents described above with respect to the server 200 illustrated in FIG. 2A or 2B may be applied to the data providing method of FIG. 5.

In operation S50, the server 200 receives identification information of a user from the ultrasound apparatus 100. The server 200 may determine whether identification information previously stored in the server 200 and the received identification information are identical to each other by comparing them with each other.

In operation S60, the server 200 transmits data including use environment information of the ultrasound apparatus 100, which corresponds to the received identification information, to the ultrasound apparatus 100.

The data which the server 200 transmits to the ultrasound apparatus 100 may include medical examination information of an examinee. In addition, when changed use environment information or newly obtained medical examination information are received from the ultrasound apparatus 100, the server 200 may store the changed use environment or the newly obtained medical examination information in correspondence to the identification information of a user, and thus may update corresponding information.

The embodiments of the present invention can be written as computer programs and can be implemented in general-use digital computers that execute the programs using a computer-readable recording medium. Examples of the computer-readable recording medium include storage media such as magnetic storage media (e.g., ROM, floppy disks, hard disks, etc.), optical recording media (e.g., CD-ROMs, or DVDs), etc.

An apparatus and method for providing data to a user of an ultrasound apparatus according to an embodiment of the present invention enables a user to easily obtain an ultrasound image, which the user has previously captured, by storing ultrasound images obtained by using the ultrasound apparatus in an external server in correspondence to each of users.

In addition, an apparatus and method for providing data to a user of an ultrasound apparatus according to an embodiment of the present invention enables previously set use environment to be applied to another ultrasound apparatus although a user does not use previously used ultrasound apparatus but use another ultrasound apparatus.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A method of providing data to a user of an ultrasound apparatus, the method comprising:
transmitting identification information input by the user to an external server;
receiving data including use environment information of the ultrasound apparatus, which corresponds to the identification information, from the external server; and
changing use environment of the ultrasound apparatus according to the received use environment information.

2. The method of claim 1, further comprising transmitting changed use environment information to the external server when the use environment of the ultrasound apparatus is changed by the user.

3. The method of claim 1, wherein the use environment information comprises at least one of information about a method of displaying an image obtained by the ultrasound apparatus, information about a method of calculating values measured by the ultrasound apparatus, and information about an user interface (UI) of the ultrasound apparatus.

4. The method of claim 1, wherein the data further comprises medical examination information of an examinee.

5. The method of claim 4, wherein the medical examination information comprises at least one of information about the examinee, an ultrasound image of the examinee, and a measured value obtained from the ultrasound image.

6. A method of providing data to a user of an ultrasound apparatus, the method comprising:
receiving identification information of a user from the ultrasound apparatus; and
transmitting data including use environment information of the ultrasound apparatus, which corresponds to the received identification information, to the ultrasound apparatus.

7. The method of claim 6, further comprising:
receiving changed use environment information of the ultrasound apparatus from the ultrasound apparatus; and
storing the changed use environment information in correspondence to the identification information of the user.

8. An apparatus for providing data to a user of an ultrasound apparatus, the apparatus comprising:
a transmission unit for transmitting identification information input by the user to an external server;
a reception unit for receiving data including use environment information of the ultrasound apparatus, which corresponds to the identification information, from the external server; and
a control unit for changing use environment of the ultrasound apparatus according to the received use environment information.

9. The apparatus of claim 8, further comprising a sensing unit for sensing whether the use environment of the ultrasound apparatus has been changed by the user,
wherein the transmission unit transmits changed use environment informaition of the ultrasound apparatus to the external server.

10. The apparatus of claim 8, wherein the use environment information comprises at least one of information about a method of displaying an image obtained by the ultrasound apparatus, information about a method of calculating values measured by the ultrasound apparatus, and information about an user interface (UI) of the ultrasound apparatus.

11. The apparatus of claim 8, wherein the data further comprises medical examination information of an examiner.

12. The apparatus of claim 11, wherein the medical examination information comprises at least one of information about the examinee, an ultrasound image of the examinee, and a measured value obtained from the ultrasound image.

13. A server for providing data to a user of an ultrasound apparatus, the server comprising:
a reception unit for receiving identification information of a user from the ultrasound apparatus; and
a transmission unit for transmitting data including use environment information of the ultrasound apparatus, which corresponds to the received identification information, to the ultrasound apparatus.

14. A computer-readable recording medium storing a computer-readable program for executing the method of any one of claims 1 through 5.

15. A computer-readable recording medium storing a computer-readable program for executing the method of claims 6 or 7.
